# EUROPEAN PATENT APPLICATION

(11) **EP 4 534 046 A1**
(43) Date of publication of application: **09.04.2025**
(21) Application number: 23814645.0
(22) Date of filing: 10.02.2023
(51) Int. Cl.: A61F 2/24

(54) **IMPLANT RELEASE DIRECTIONAL MARKING DEVICE, IMPLANT CONVEYING SYSTEM AND WORKING METHOD THEREFOR**

(30) Priority: 30.05.2022 CN 202210605616
(71) Applicant: Shanghai Trulive Medtech Co., Ltd., Shanghai 201206 (CN); Jiangsu Trulive Medtech Co., Ltd., Nantong, Jiangsu 226400 (CN)
(72) Inventor: HAN, Tianyu, Shanghai 201206 (CN); LIU, Xiang, Shanghai 201206 (CN); WEI, Yongqiang, Shanghai 201206 (CN)
(74) Representative: Patentship Patentanwaltsgesellschaft mbH
(86) International application number: PCT/CN2023/075381
(87) International publication number: WO 2023/231446

(57) **Abstract**

An implant release directional marking device, an implant delivery system and a method of operation thereof are provided. The implant release directional marking device includes a marking structure having a marking tab (20) movably mounted on a wall of an outer tube (10). When out of use, the marking tab (20) is embedded in the wall of the outer tube (10). When in use, a proximal end of the marking tab (20) is tilted in a direction away from an axis of the outer tube (10). This allows for controlled directional marking once the implant delivery system enters the body, thereby enabling control of the circumferential angle of the valve stent. This ensures that the profiled valve stent can match well with the native annulus, allowing the implant delivery system to more accurately release the valve prosthesis.

## Description

### TECHNICAL FIELD

The present invention relates to the technical field of medical devices, and particularly to implant release directional marking devices, implant delivery systems and methods of operation thereof.

### BACKGROUND

The heart has four chambers: the left atrium (LA) and left ventricle (LV) both at the left side of the heart; and the right atrium (RA) and right ventricle (RV) at the right side of the heart. The ventricular inflow tracts are formed between the atria and ventricles, the left ventricular outflow tract is formed between the left ventricle and aorta, and the right ventricular outflow tract is formed between the right ventricle and pulmonary artery. Throughout cardiac cycles, pumping of the left atrium and ventricle generally occurs in synchrony with the pumping of the right atrium and ventricle. The human heart has four valves: the aortic valve connecting the left ventricle to the aorta; the pulmonary valve connecting the right ventricle to the pulmonary artery; the mitral valve connecting the left atrium to the left ventricle; and the tricuspid valve connecting the right atrium to the right ventricle. All these valves function like one-way valves which allow blood flow in the forward direction but prevent the backward flow of blood. At the beginning of ventricular filling (i.e., diastole), the aortic and pulmonary valves close to prevent blood from flowing from the arteries back into the ventricles. Soon after that, the mitral and tricuspid valves open to allow unimpeded flow from the atria into the respective ventricles. Shortly after ventricular systole (i.e., ventricular emptying), the tricuspid and mitral valves close normally to form a seal against backflow from the ventricles into the respective atria.

The human heart has very complex anatomy. In particular, the mitral valve is more anatomically complex than the aortic valve since the mitral annulus has an irregular shape. This, coupled with the fact that there are many chordae tendineae in the ventricles, would create great challenges to the implantation and positioning of a prosthetic valve. Therefore, transcatheter mitral valve replacement (TMVR) relies on catheter-based intervention for delivery of a prosthetic valve, which has been crimped into a delivery system outside the body of a patient, to the annulus of the native mitral value within the body through a vascular or transapical access. After that, the prosthetic valve is released and anchored in the mitral valve annulus to replace the native valve. Compared with the surgical approach, TMVR eliminates the use of a cardiopulmonary bypass machine, causes less trauma, allows fast patient recovery and provides significant improvements in postoperative hemodynamic indices. However, due to the complex anatomy of the mitral valve, which is essentially characterized by the unique saddle-like shape of the annulus, the irregularly-shaped leaflets, a large diameter of the annulus and absence of calcification thereof in most cases, complex subvalvular structures including chordae tendineae and papillary muscles, the complex adjacent tissues (i.e., the coronary arteries and left ventricular outflow tract), and the thin, fragile left atrial wall, most stents of such prosthetic valves are designed as profiled ones.

Based on a profiled stent and an irregular mitral valve, it is extremely important to achieve a match between the profiled stent and the mitral valve. How well they match determines whether the prosthetic valve can function as it is intended to. A poor match may give rise to problems, such as paravalvular leakage, loose anchoring and deterioration of the heart function. From the experiments performed so far on locating a profiled stent that is immediately released is based upon a CT image, while there are profiled stents used as a basis for determining position, the stent's surface is largely covered with fabric, leading to CT images that only display a blurry stent, making it challenging to determine the location of the stent from the CT images, particularly, when the stent has just been released from the delivery system, with the fabric on the surface of the stent being compressed together.

### SUMMARY OF THE INVENTION

It is an object of the present invention to provide implant release directional marking devices, implant delivery systems and methods of operation thereof, which overcome the problem of inaccurate locating of a profiled stent during its release, which leads to an inaccurate match between the profiled stent and a mitral valve structure.

The above object is attained by an implant release directional marking device according to the present invention, which is arranged on a wall of a distal end of an outer tube of a catheter assembly. The implant release directional marking device includes a marking structure having a marking tab movably mounted on the wall of the outer tube, wherein the marking tab is embedded in the wall of the outer tube when out of use, and wherein a proximal end of the marking tab is tilted in a direction away from an axis of the outer tube when in use.

Optionally, the distal end of the outer tube may be provided with a first slot, the marking tab is rotatably mounted in the first slot, a shape of the marking tab matches a shape of the first slot.

Optionally, the marking structure may comprise a distal lug and a first seat, which are arranged on the same surface of the marking tab, the distal lug located at a distal end of the first seat and arranged at a distal end of the marking tab, the distal lug configured for passage of a pull cord therethrough.

Optionally, the implant release directional marking device may further comprise a movably connecting member and a restoring member, the movably connecting member coupled to each of the outer tube, the restoring member and the first seat of the marking structure, the restoring member configured to provide the marking tab with a reset torque to ensure that the marking tab is maintained at, or restored to, the out-of-use configuration.

Optionally, the movably connecting member may comprise a second seat and a rotating shaft.

The second seat may be fixed to an inner surface of the outer tube in the vicinity of the first slot in the outer tube, and an axis of the first seat coincides with an axis of the second seat.

The rotating shaft may be fixedly coupled at its first end within the first seat and rotatably disposed at its second end within the second seat, wherein the restoring member is sleeved over the rotating shaft, the restoring member is fixedly coupled to the second seat at its first end and to the rotating shaft at its second end. Alternatively, the rotating shaft may be rotatably disposed at its first end within the first seat and fixedly coupled at its second end within the second seat, wherein the restoring member is sleeved over the rotating shaft, the restoring member is fixedly coupled to the first seat at its first end and to the rotating shaft at its second end.

In a second aspect of the present invention, there is provided an implant delivery system comprising a catheter assembly and the implant release directional marking device as defined above. The implant release directional marking device is located on a wall of a distal end of an outer tube of the catheter assembly, and the marking tab is embedded in the wall of the outer tube when out of use and a proximal end of the marking tab is tilted in a direction away from an axis of the outer tube when in use..

In a third aspect of the present invention, there is provided a method of operation of the implant delivery system as defined above, which comprises:
when a distal end of the catheter assembly reaches the vicinity of a release site, proximally pulling a pull cord to clockwise rotate the marking tab, thereby causing a proximal end of the marking tab to tilt in a direction away from the axis of the outer tube; and
when the distal end of the catheter assembly reaches the release site, releasing the pull cord to allow the marking tab to counterclockwise rotate, thereby causing the marking tab to return to the first slot under an effect of a restoring structure.

In a fourth aspect of the present invention, there is provided an implant release directional marking device, which is arranged on a catheter assembly. The implant release directional marking device comprises a balloon provided on an outer surface of a distal end of an outer tube of the catheter assembly. The balloon comprises a collapsed configuration when out of use, in which it is collapsed on the outer surface. The balloon comprises an expanded configuration when in use, in which it is a protrusion structure located on the outer surface.

Optionally, the implant release directional marking device may further comprise a liquid supply path disposed within the outer tube, a distal end of the liquid supply path communicates with the balloon, a proximal end of the liquid supply path configured to be connected to a liquid inlet to allow a liquid to be introduced into the balloon through the liquid supply path.

In a fifth aspect of the present invention, there is provided an implant delivery system comprising a delivery handle, a catheter assembly and the implant release directional marking device as defined above.

The liquid supply path is formed between inner and outer tubes of the catheter assembly. The balloon is provided on an outer surface of a distal end of the outer tube of the catheter assembly. A liquid inlet is provided at a distal end of the delivery handle and is configured for supply of a liquid to the balloon through the liquid supply path, which allows the balloon to comprise a collapsed configuration and an expanded configuration, wherein when out of use, the balloon is in the collapsed configuration and is collapsed on the outer surface, wherein when in use, the balloon is in the expanded configuration and is a protrusion structure located on the outer surface.

In a sixth aspect of the present invention, there is provided a method of operation of the implant delivery system as defined above, which comprises:
when a distal end of the catheter assembly reaches the vicinity of a release site, liquid is introduced into the balloon from the liquid inlet through the liquid supply path, causing the balloon to be in an expanded configuration; and
when the distal end of the catheter assembly reaches the release site, the liquid is evacuated from the balloon to the liquid inlet through the liquid supply path, causing the balloon to be in a collapsed configuration.

Compared with the prior art, the present invention has the benefits as follows:
It provides an implant release directional marking device, an implant delivery system and a method of operation thereof. The implant release directional marking device includes a marking structure having a marking tab movably mounted on a wall of an outer tube. The marking tab is embedded in the wall of the outer tube when out of use, and a proximal end of the marking tab is tilted in a direction away from an axis of the outer tube when in use. This allows for controlled directional marking once the implant delivery system enters the body, thereby enabling control of the circumferential angle of the valve stent. This ensures that the profiled valve stent can match well with the native annulus, allowing the implant delivery system to more accurately release the valve prosthesis (i.e., the valve stent).

### BRIEF DESCRIPTION OF THE DRAWINGS

Figs. 1 to 3 are schematic perspective views of an implant release directional marking device according to a first embodiment of the present invention.
Fig. 4 is a schematic partial cross-sectional view of the implant release directional marking device according to the first embodiment of the present invention.
Fig. 5 shows a simplified schematic of the implant release directional marking device according to the first embodiment of the present invention.
Figs. 6a to 6d schematically illustrate an implant delivery system in operation according to the first embodiment of the present invention.
Fig. 7 is a schematic structural view of an implant delivery system according to a second embodiment of the present invention.
Figs. 8 to 9 are schematic structural views of an implant release directional marking device according to the second embodiment of the present invention.

In these figures,
10, an outer tube; 11, a first slot; 20, a marking tab; 21, a distal lug; 22, a first seat; 31, a second seat; 32, a rotating shaft; 33, a restoring member; 41, a liquid inlet; 42, a liquid supply path; and 43, a balloon.

### DETAILED DESCRIPTION

As discussed in the Background section, blurriness may be encountered when locating a profiled stent during its release on the basis of its own body. From attempts at addressing this problem, the inventors have found that once a (profiled) valve stent is crimped in an implant delivery system, its location with respect to the implant delivery system is relatively definite. Accordingly, it would be feasible to determine the location of the valve stent from a visible feature in a CT image of the implant delivery system.

In view of this, in a first aspect of the present invention, there is provided an implant release directional marking device, which is arranged on a wall of an outer tube of a catheter assembly at a distal end thereof. The implant release directional marking device includes a marking structure having a marking tab movably mounted on the wall of the outer tube, when out of use, the marking tab is embedded in the wall of the outer tube, and when in use, the proximal end of the marking tab is tilted in a direction away from an axis of the outer tube.

In a second aspect, there is provided an implant delivery system including a catheter assembly and the implant release directional marking device of the first aspect. The implant release directional marking device is located on a wall of an outer tube of the catheter assembly at a distal end thereof, when out of use, the marking tab is embedded in the wall of the outer tube, and when in use, the proximal end of the marking tab is tilted in a direction away from an axis of the outer tube.

In a third aspect, there is provided a method of operation of the implant delivery system of the second aspect, which includes:
when a distal end of the catheter assembly reaches the vicinity of a release site, proximally pulling a pull cord to clockwise rotate the marking tab, thereby causing a proximal end of the marking tab to tilt in a direction away from the axis of the outer tube ; and
when the distal end of the catheter assembly reaches the release site, releasing the pull cord to allow the marking tab to counterclockwise rotate, thereby causing the marking tab to return to the first slot under an effect of an restoring structure.

In a fourth aspect, there is provided an implant release directional marking device, which is arranged on a catheter assembly. The marking tab device includes a balloon provided on an outer surface of an outer tube of the catheter assembly at a distal end thereof. The balloon comprises a collapsed configuration when out of use, in which it is collapsed on the outer surface. The balloon comprises an expanded configuration when in use, in which it is a protrusion structure located on the outer surface.

In a fifth aspect, there is provided an implant delivery system, including a delivery handle, a catheter assembly and the implant release directional marking of the fourth aspect.

The liquid supply path is formed between inner and outer tubes of the catheter assembly. The balloon is provided on an outer surface of the outer tube of the catheter assembly at a distal end thereof. A liquid inlet is provided at a distal end of the delivery handle and is configured for supply of a liquid to the balloon through the liquid supply path, allowing the balloon to comprise a collapsed configuration and an expanded configuration, wherein when out of use, the balloon is in the collapsed configuration and is collapsed on the outer surface, wherein when in use, the balloon is in the expanded configuration and is a protrusion structure located on the outer surface.

In a sixth aspect, there is provided a method of operation of the implant delivery system of the fifth aspect, which includes:
when a distal end of the catheter assembly reaches the vicinity of a release site, liquid is introduced into the balloon from the liquid inlet through the liquid supply path, causing the balloon to be in a expanded configuration; and
when the distal end of the catheter assembly reaches the release site, the liquid is evacuated from the balloon to the liquid inlet through the liquid supply path, causing the balloon to be in a collapsed configuration.

Implant release directional marking devices, implant delivery systems and methods of operation thereof, which embody the present invention, will be described in greater detail below. The invention will be described in greater detail below with reference to the accompanying drawings, which present preferred embodiments thereof. It would be appreciated that those skilled in the art can make changes to the invention disclosed herein while still obtaining the beneficial results thereof. Therefore, the following description shall be construed as being intended to be widely known by those skilled in the art rather than as limiting the invention.

For the sake of clarity, not all features of an actual implementation are described in this specification. In the following, description and details of well-known functions and structures are omitted to avoid unnecessarily obscuring the invention. It should be appreciated that in the development of any such actual implementation, numerous implementation-specific decisions must be made to achieve specific goals of the developers, such as compliance with system-related and business-related constrains, which may vary from one implementation to another. Moreover, it should be appreciated that such a development effort might be complex and time consuming, but would nevertheless be a routine undertaking for those of ordinary skill in the art.

In the following paragraphs, the present invention will be described in greater detail by way of examples with reference to the accompanying drawings. Advantages and features of the present invention will become more apparent from the following description. Note that the figures are provided in a very simplified form not necessarily drawn to exact scale for the only purpose of helping in describing the embodiments in a convenient and clear way. As used herein, the term "or" is generally employed in the sense of "and/or", unless the context clearly dictates otherwise. As used herein, the terms "inner", "outer" and similar terms are merely illustrative and do not represent the only implementation possible. As used herein, the terms "distal end" and "proximal end" are employed to describe orientations, positions and directions of components of a medical device or actions thereon relative to one another, as viewed by a surgeon who is operating the medical device. "Proximal end" is usually used to describe an end closer to the operator, in contrast to "distal end" being usually used to describe an end farther away from the operator, during normal operation of the medical device, although these terms are not intended to be limited to being used in such a way.

### Embodiment 1

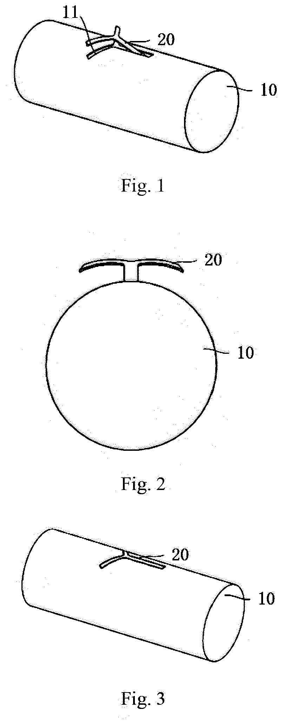

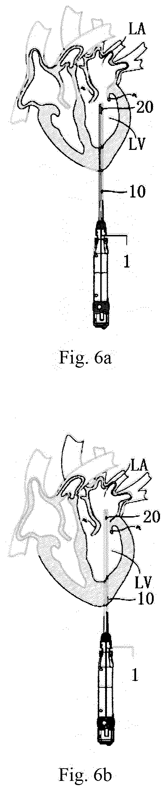

Figs. 1 to 3 are schematic perspective views of an implant release directional marking device according to a first embodiment of the present invention. Figs. 6a to 6d schematically illustrate an implant delivery system in operation according to this first embodiment. Referring to Figs. 6a to 6d, in conjunction with Figs. 1 to 3, the implant delivery system of this embodiment includes a delivery handle, a catheter assembly and the implant release directional marking device.

The catheter assembly is fixedly coupled to the delivery handle and a distal end of the catheter assembly extends beyond a distal end of the delivery handle. The catheter assembly carries a valve stent, e.g., a profiled valve stent, at its distal end. The implant release directional marking device is partially movably disposed on a wall of a distal end of the catheter assembly. In detail, when out of use, the implant release directional marking device is integrated, i.e., embedded, in the catheter assembly. When in use, the implant release directional marking device is partially tilted in a direction away from an axis of the catheter assembly to mark direction of the valve stent during its release, thereby controlling circumferential angle of the valve stent in order to enable a well match between the valve stent and the native annulus, allowing the implant delivery system to more accurately release the valve stent. The implant release directional marking device can be integrated into the implant system without compromising its capabilities at all. It is a controllable marking tab, which is simple in structure and highly reliable and can provide accurate locating.

The catheter assembly includes, an outer tube 10, an inner tube and a guidewire tube, which is sequentially sleeved one over another from the outside inwards. That is, the outer tube 10 is sleeved over the inner tube, and the inner tube in turn is sleeved over the guidewire tube. The outer tube 10 provides a first slot 11 at its distal end, which extends through a wall of the outer tube 10 and brings the outside of the outer tube 10 into communication with the interior thereof. The first slot 11 is configured for disposal of a potion of the implant release directional marking device therein.

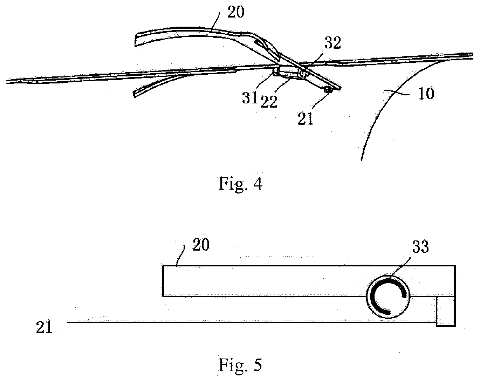

Fig. 4 is a schematic partial cross-sectional view of implant release directional marking device according to this first embodiment. Fig. 5 shows a simplified schematic of implant release directional marking device according to this embodiment. Referring to Figs. 4 to 5, in conjunction with Figs. 1 to 3, the implant release directional marking device includes a marking structure, a movably connecting member and a restoring member 33. The movably connecting member is connected to each of the outer tube 10, the restoring member 33 and the marking structure. In detail, a portion of the movably connecting member may be fixed to an inner surface of the outer tube 10, and another portion of the movably connecting member may be movably connected to the marking structure. In this case, the restoring member 33 is connected to both the marking structure and the other portion of the movably connecting member. Alternatively, a portion of the movably connecting member may be movably connected to the inner surface of the outer tube 10, and another portion of the movably connecting member may be fixed to the marking structure. In this case, the restoring member 33 is connected to both the marking structure and the portion of the movably connecting member. Still alternatively, a portion of the movably connecting member may be fixed to the inner surface of the outer tube 10, and another portion of the movably connecting member may be rotatably connected to the marking structure. In this case, the restoring member 33 is connected to both the marking structure and the other portion of the movably connecting member. Yet still alternatively, a portion of the movably connecting member may be rotatably connected to the inner surface of the outer tube 10, and another portion of the movably connecting member may be fixed to the marking structure. In this case, the restoring member 33 is connected to both the marking structure and the portion of the movably connecting member.

The restoring member 33 is used to provide a reset torque to the marking structure, so that the marking structure can maintain at or restored to its state when not in use. In this embodiment, the restoring member 33 may be, for example, a restoring spring with two free ends. One of the free ends of the restoring spring is fixedly coupled to the marking structure, and the other free end is fixedly coupled to the movably connecting member.

The marking structure includes a marking tab 20, a distal lug 21 and a first seat 22. The marking tab 20 is disposed in the first slot 11 so as to remain integral with the wall of the outer tube 10 when out of use. That is, the marking tab 20 is embedded in the first slot 11. The distal lug 21 and the first seat 22 are arranged on the same side of the marking tab 20, the first seat 22 is located around the middle of the marking tab 20, the distal lug 21 is located at a distal end of the marking tab 20, the first seat 22 is coupled to both the movably connecting member and the restoring member 33, the distal lug 21 is configured to be connected to a distal end of a pull cord, so that when the pull cord is pulled from the proximal end thereof, the distal end of the marking tab 20 can rotate clockwise into the outer tube 10, while the proximal end of the marking tab 20 can rotate clockwise to the outside of the outer tube 10 (i.e., tilt), allowing for directional marking of the valve stent during use .

A shape of the marking tab 20 matches a shape of the first slot 11 so that it can be received in and rotate within the first slot 11. Specifically, the marking tab 20 may overall comprise the shape of a letter, such as "Y". Alternatively, it may also comprise a regular shape, such as that of an ellipse or star. This shape imparts an obvious structural characteristic to the marking tab 20, making it easy to recognize in a CT image. Of course, the marking tab 20 may also have any other shape that can impart an obvious structural characteristic. The marking tab 20 may define the same axial direction as the outer tube 10. The marking tab 20 may be made of, for example, a biocompatible polymer material. Examples of this may include, but are not limited to, ultra-high molecular weight polyethylene (UHMWPE) and polytetrafluoroethylene (PTFE).

The lug is shaped like a ring, for example, and configured for passage of the pull cord therethrough. The first seat 22 is, for example, a cylindrical tube open at both ends, which comprises an axial direction perpendicular to an axial direction of the marking tab 20. The first seat 22 is sleeved over a portion of the movably connecting member.

The movably connecting member includes a second seat 31 and a rotating shaft 32. The second seat 31 is also a cylindrical tube open at both ends, which is fixed to the inner surface of the outer tube 10 in the vicinity of the first slot 11, and an axis of the first seat 22 coincides with an axis of the second seat 31. In one example, one end of the rotating shaft 32 is fixed within the first seat 22, and the other end of the rotating shaft 32 is rotatably arranged within the second seat 31. In this case, the restoring member 33 is sleeved over the rotating shaft 32, the restoring member 33 is fixedly coupled to the second seat 31 at one end and to the rotating shaft 32 at the other end. With this arrangement, retracting the distal end of the marking tab 20 by pulling the pull cord can rotate the rotating shaft 32 and hence the first seat 22 to tilt the proximal end of the marking tab 20, creating an angle between the axis of the marking tab 20 and that of the outer tube 10. At the same time, the restoring member 33 applies a reset torque to the rotating shaft 32, which tends to restore the marking tab 20 to the out-of-use configuration in which the marking tab 20 is embedded in the first slot 11 and is integral with the outer tube 10, with the axis of the marking tab 20 running parallel to the axis of the outer tube 10.

In another example, one end of the rotating shaft 32 is rotatably arranged within the first seat 22, and the other end of the rotating shaft 32 is fixed to the second seat 31. In this case, the restoring member 33 is sleeved over the rotating shaft 32, and the restoring member 33 is fixedly coupled to the first seat 22 at one end and to the rotating shaft 32 at the other end. With this arrangement, retracting the distal end of the marking tab 20 by pulling the pull cord can rotate the first seat 22 to tilt the proximal end of the marking tab 20, creating an angle between the axis of the marking tab 20 and the axis of the outer tube 10. At the same time, the restoring member 33 applies a reset torque to the first seat 22, which tends to restore the marking tab 20 to the out-of-use configuration in which the marking tab 20 is embedded in the first slot 11 and is integral with the outer tube 10, with the axis of the marking tab 20 running parallel to that of the outer tube 10.

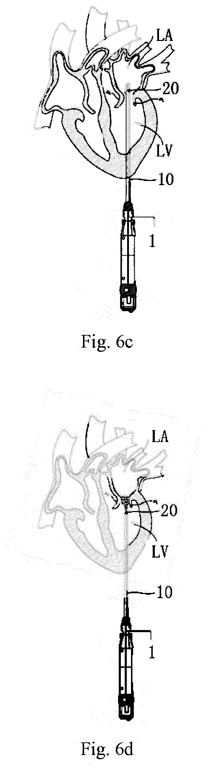

A method of operating the implant delivery system includes:
as shown in Fig. 6a, when the implant delivery system is prepared to enter the body, the marking tab 20 is embedded in the first slot 11 and is integrated with the outer tube 10, which does not affect the catheter assembly from entering the human body. As shown in Figure 6b, once the implant delivery system enters the heart, the distal end of the catheter assembly first reaches the vicinity of the release position. At this stage, the profiled valve stent has not yet been released, and in order to determine the circumferential position of the catheter assembly and the valve stent, a pull cord is pulled from the proximal end, causing the marking tab 20 to rotate clockwise around the rotational axis 32. This results in the proximal end of the marking tab 20 tilting outward away from the outer surface of the outer tube 10 (for example, as shown in Fig. 6c, the Y-shaped structure can be readily identified in a CT image); subsequently, optionally finely tuning an axial angle of the implant delivery system, locking the axial direction, and then releasing the pull cord to allow the restoring member 33 to counterclockwise rotate the marking tab 20 to return to the out-of-use configuration; after that, as shown in Fig. 6d, finely tuning the implant delivery system to the release site by axially moving it forth or back and then releasing the valve stent; and after the release is completed, withdrawing the implant delivery system from the human body, with the marking tab 20 being received within the first slot 11 without affecting the withdrawal of the outer tube 10.

### Embodiment 2

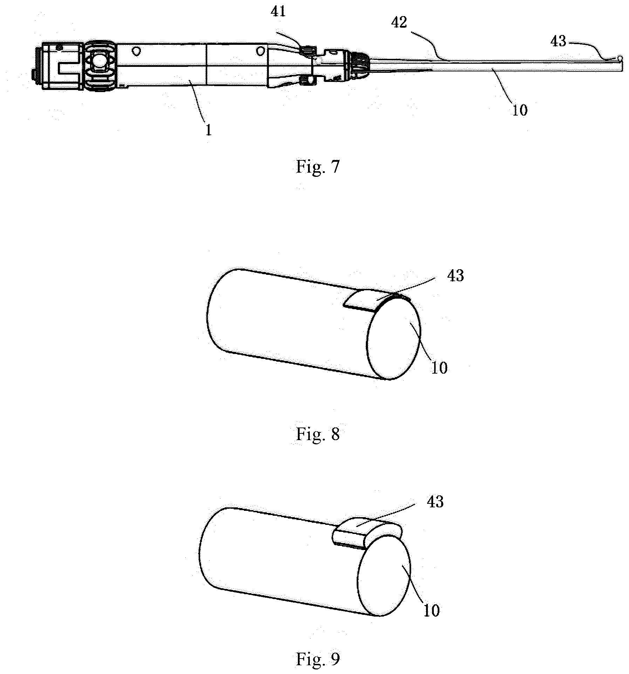

Differing from the pull cord-based implant release directional marking device of the first embodiment, a second embodiment of the present invention provides an implant release directional marking device, in which a balloon 43 is employed. Specifically, Fig. 7 shows a schematic structural view of the implant delivery system according to this second embodiment, and Figs. 8 to 9 are schematic perspective views of the implant release directional marking according to this embodiment. As shown in Figs. 7 to 9, the implant release directional marking device of this embodiment includes a liquid inlet 41, a liquid supply path 42 and the balloon 43. The liquid inlet 41 is provided at a distal end of a delivery handle, and the balloon 43 is disposed on an outer surface of a distal end of an outer tube 10. The liquid supply path 42 runs between the outer tube 10 and an inner tube. One end of the liquid supply path 42 communicates with the liquid inlet 41, and the other end communicates with the balloon 43. Specifically, the wall of the outer tube 10 is provided therein with a second slot, through which an opening of the balloon 43 is passed and brought into communication with the other end of the liquid supply path 42.

The liquid inlet 41 is configured for connection with a liquid supply device, and the balloon 43 can be inflated or deflated by controlling an amount of liquid introduced into the balloon 43 through the liquid inlet 41. Accordingly, the balloon 43 can have a collapsed configuration and an expanded configuration. Specifically, when in the collapsed configuration, there is no liquid introduced from the liquid supply device through the liquid inlet 41 and the liquid supply path 42 into the balloon 43, the balloon 43 is collapsed on the outer surface of the outer tube 10, making the balloon 43 and the wall integral. The balloon 43 has a thin, ring-shaped profile that does not affect the insertion or removal of the catheter assembly, nor does it affect the axial movement of the inner tube. The balloon 43 can comprise the expanded configuration as a result of introducing a liquid from the liquid supply device through the liquid inlet 41 and the liquid supply path 42 into the balloon 43. In this configuration, the balloon 43 can be clearly seen in a CT image. When expanded, the balloon 43 may comprise a ball shape or teardrop shape, and its convexly curved side contour can be clearly identified in a CT image. Of course, the balloon 43 may also be designed to have any other shape when in the expanded configuration, which allows it to be clearly observed. The balloon 43 may be made of a biocompatible polymer material, such as PTFE or the like.

A method of operating the implant delivery system includes:
when the implant delivery system is prepared to enter the body, the balloon 43 is not filled with fluid, causing it to be collapsed on the outer surface of the outer tube 10 and to integrate with the wall of the outer tube 10. In this state, the balloon 43 is in a collapsed condition and does not affect the catheter assembly from entering the human body; after the implant delivery system enters the heart, the distal end of the catheter assembly reaches the vicinity of the release position first, at this point, the profiled valve stent has not yet been released. To determine the circumferential position of the catheter assembly and the valve stent, liquid is introduced through the liquid inlet 41, causing the balloon 43 to inflate and expand (i.e., the balloon 43 is in an expanded configuration), and its convexly curved contour can be readily recognized in a CT image; subsequently, optionally finely tuning an axial angle of the implant delivery system, locking the axial direction, and then evacuating the liquid to collapse the balloon 43 onto the outer surface of the outer tube 10 (i.e., to restore it to the collapsed configuration); after that, finely tuning the implant delivery system to the release site by axially moving it forth or back and releasing the valve stent; and after the release is completed, withdrawing the implant delivery system from the human body, with the balloon 43 being maintained collapsed on the outer surface of the outer tube 10 without affecting the withdrawal of the outer tube 10.

In summary, the present invention provides implant release directional marking devices, implant delivery systems and methods of operation thereof. The implant release directional marking device is designed integral with the implant delivery system, and features a small and compact structure that does not affect the system's original delivery functions. The marking structure of the implant release directional marking device is controllable, when not in use, it does not affect the operation of the implant delivery system, when in use, the device's protrusion or expanding features are utilized to make it identifiable in CT imaging. This ensures that the implant release directional marking device is simple in structure and highly reliable and can provide accurate locating.

Further, it is to be understood that, as used herein, the terms "first", "second" and the like are only meant to distinguish various components, elements, steps, etc. from each other and are not intended to indicate logical or sequential orderings thereof, unless otherwise indicated or specified.

It would be appreciated that while the invention has been described above with reference to preferred embodiments thereof, it is not limited to these embodiments. In light of the above teachings, any person familiar with the art may make many possible modifications and variations to the disclosed embodiments or adapt them into equivalent embodiments, without departing from the scope of the invention. Accordingly, it is intended that any and all simple variations, equivalent alternatives and modifications made to the foregoing embodiments based on the substantive disclosure of the invention without departing from the scope thereof fall within the scope.

## Claims

1. An implant release directional marking device, arranged on a wall of a distal end of an outer tube of a catheter assembly, wherein the implant release directional marking device comprises a marking structure having a marking tab movably mounted on the wall of the outer tube, wherein the marking tab is embedded in the wall of the outer tube when out of use, and wherein a proximal end of the marking tab is tilted in a direction away from an axis of the outer tube when in use.

2. The implant release directional marking device of claim 1, wherein the distal end of the outer tube is provided with a first slot, wherein the marking tab is rotatably mounted in the first slot, and wherein a shape of the marking tab matches a shape of the first slot.

3. The implant release directional marking device of claim 1, wherein the marking structure comprises a distal lug and a first seat that are arranged on a same surface of the marking tab, wherein the distal lug is located at a distal end of the first seat and is arranged at a distal end of the marking tab, and wherein the distal lug is configured for passage of a pull cord therethrough.

4. The implant release directional marking device of claim 1, further comprising a movably connecting member and a restoring member, wherein the movably connecting member is coupled to each of the outer tube, the restoring member and a first seat of the marking structure, and wherein the restoring member is configured to provide the marking tab with a reset torque to ensure that the marking tab is maintained at, or restored to, an out-of-use configuration.

5. The implant release directional marking device of claim 4, wherein the movably connecting member comprises a second seat and a rotating shaft,
wherein the second seat is fixed to an inner surface of the outer tube in a vicinity of a first slot in the outer tube, and wherein an axis of the first seat coincides with an axis of the second seat, and
wherein a first end of the rotating shaft is fixedly coupled to the first seat and a second end of the rotating shaft is rotatably disposed within the second seat, wherein the restoring member is sleeved over the rotating shaft, and wherein a first end of the restoring member is fixedly coupled to the second seat, and a second end of the restoring member is fixedly coupled to the rotating shaft; or wherein a first end of the rotating shaft is rotatably disposed within the first seat and a second end of the rotating shaft is fixedly coupled to the second seat, wherein the restoring member is sleeved over the rotating shaft, and wherein a first end of the restoring member is fixedly coupled to the first seat and a second end of the restoring member is fixedly coupled to the rotating shaft.

6. An implant delivery system, comprising a catheter assembly and the implant release directional marking device of any one of claims 1 to 5, wherein the implant release directional marking device is located on a wall of a distal end of an outer tube of the catheter assembly, wherein the marking tab is embedded in the wall of the outer tube when out of use, and wherein a proximal end of the marking tab is tilted in a direction away from an axis of the outer tube when in use.

7. A method of operation of the implant delivery system of claim 6, comprising:
when a distal end of the catheter assembly reaches a vicinity of a release site, proximally pulling a pull cord to clockwise rotate the marking tab, thereby causing a proximal end of the marking tab to tilt in a direction away from the axis of the outer tube; and
wherein when the distal end of the catheter assembly reaches the release site, releasing the pull cord to allow the marking tab to counterclockwise rotate, thereby causing the marking tab to return to the first slot under an effect of an restoring structure.

8. An implant release directional marking device, arranged on a catheter assembly, wherein the implant release directional marking device comprises a balloon provided on an outer surface of a distal end of an outer tube of the catheter assembly, wherein the balloon comprises a collapsed configuration when out of use, in which the balloon is collapsed on the outer surface, and wherein the balloon comprises an expanded configuration when in use, in which the balloon is a protrusion structure located on the outer surface.

9. The implant release directional marking device of claim 8, further comprising a liquid supply path disposed within the outer tube, wherein a distal end of the liquid supply path communicates with the balloon, and a proximal end of the liquid supply path is configured to be connected to a liquid inlet to allow a liquid to be introduced into the balloon through the liquid supply path.

10. An implant delivery system, comprising a delivery handle, a catheter assembly and the implant release directional marking device of claim 8 or 9,
wherein the liquid supply path is disposed between an inner tube and the outer tube of the catheter assembly, wherein the balloon is provided on an outer surface of a distal end of the outer tube of the catheter assembly, wherein a liquid inlet is provided at a distal end of the delivery handle and is configured for supply of a liquid to the balloon through the liquid supply path, allowing the balloon to comprise a collapsed configuration and an expanded configuration, wherein when out of use, the balloon is in the collapsed configuration and is collapsed on the outer surface, wherein when in use, the balloon is in the expanded configuration and is a protrusion structure located on the outer surface.

11. A method of operation of the implant delivery system of claim 10, comprising:
when a distal end of the catheter assembly reaches a vicinity of a release site, liquid is introduced into the balloon from the liquid inlet through the liquid supply path, causing the balloon to be in an expanded configuration; and
when the distal end of the catheter assembly reaches the release site, the liquid is evacuated from the balloon to the liquid inlet through the liquid supply path, causing the balloon to be in a collapsed configuration.
